# EUROPEAN PATENT SPECIFICATION

(11) **EP 3 009 184 B1**
(45) Date of publication and mention of the grant of the patent: **01.05.2019**
(21) Application number: 13886736.1
(22) Date of filing: 13.06.2013
(51) Int. Cl.: B01F 3/04, A61L 9/18, B01F 5/02, B01F 5/20, B08B 3/10, C02F 1/50, C02F 1/72, C02F 1/76, C02F 1/78, D06F 35/00

(54) **METHOD AND DEVICE FOR GENERATING MICRO AND NANO BUBBLES**
VERFAHREN UND VORRICHTUNG ZUR ERZEUGUNG VON MIKRO- UND NANOBLASEN
PROCÉDÉ ET DISPOSITIF DE PRODUCTION DE MICROBULLES ET DE NANOBULLES

(43) Date of publication of application: 20.04.2016
(73) Proprietor: Sigma-Technology Inc., Hitachinaka-shi, Ibaraki 312-0011 (JP)
(72) Inventor: TACHIBANA, Yoshiaki, Hitachinaka-shi Ibaraki 312-0011 (JP); TACHIBANA, Kousuke, Hitachinaka-shi Ibaraki 312-0011 (JP); HARADA, Kaoru, Hitachinaka-shi Ibaraki 312-0011 (JP); SASAJIMA, Souzou, Hitachinaka-shi Ibaraki 312-0011 (JP); TAMAHASHI, Kunihiro, Hitachinaka-shi Ibaraki 312-0011 (JP); HONMA, Kyoko, Hitachinaka-shi Ibaraki 312-0011 (JP); MATUMOTO, Yuuki, Hitachinaka-shi Ibaraki 312-0011 (JP)
(74) Representative: Mewburn Ellis LLP
(86) International application number: PCT/JP2013/066902
(87) International publication number: WO 2014/199525

(56) References cited:
- WO-A1-2011/121631
- JP-A- S62 295 663
- JP-A- S62 295 663
- JP-A- 2000 262 876
- JP-A- 2000 325 767
- JP-A- 2002 018 253
- JP-A- 2007 038 067
- JP-A- 2008 030 002
- JP-A- 2008 030 002
- JP-A- 2010 274 254
- JP-A- 2012 236 151
- JP-A- 2012 236 151
- US-A- 4 994 242
- US-A1- 2003 230 122
- US-A1- 2009 201 761

## Description

### {TECHNICAL FIELD}

The present invention relates to a method and an apparatus for generating micro-nano bubbles, wherein the method and the apparatus use water hammering.

### {BACKGROUND OF THE INVENTION}

Washing or sterilizing by micro-nano bubbles is a method that uses only water, air, and additives of a trace quantity, offering a reduced environmental load. Due to this, such method have attracted attention as an alternative to a conventional method for washing or sterilizing that uses materials like detergents and chemicals. In addition, because of such method being highly safe, application as a sterilization method for vegetables and foods has been studied. Conventional methods for generating micro-nano bubbles have been known in three fashions: the gas-liquid two-phase swirl flow method, the venturi tube method, and the pressure dissolution method. (For example, refer to Patent Literatures 1 and 2 for the gas-liquid two-phase swirl flow method and the pressure dissolution method.)

Such conventional methods are however not satisfactory because the number of micro-nano bubbles generated by each of such methods is still not large enough. Although each of such conventional three methods can easily produce micro-bubble water, nucleating agent like base and magnesium must be added to the micro-nano water for generating a sufficient number of micro-nano bubbles. Addition of a nucleating agent has been a major obstacle in expansion of application to washing and sterilization of such as semiconductor devices and food. At present however, it is very difficult to generate a large amount of micro-nano bubbles using pure water.

Pumps of various types are employable as the driving pump in an apparatus that generates bubbles using water, air, and additives of a trace quantity. However, washing and sterilization of semiconductor devices and food require that all the pertinent apparatus components including the driving pump should operate without causing metal contamination. For example, when devices such as semiconductor wafers are to be washed without metal contaminations, all the wetted parts of pumps to be used in an apparatus for generating micro-nano bubbles should be made of those which do not generate any metal ions; and further, such pumps must operate stably at a discharge pressure of 0.3 to 0.6 MPa.

In consideration for these, the inventors of the present invention have proposed an apparatus that employs a compressed-air driven bellows-cylinder pump as a pump that feeds liquid without using rotating movements (Patent Literatures 3 and 4). All the wetted parts of the proposed pump are made of fluorine resin to avoid the feared metal contamination that will occur in a rotating type pump. To achieve the goal of performing a clean washing without the influence of contamination, technical development is desired on application of plastic by use of such as fluorine resin to all the related constituent units for generating micro-bubbles, including not only pumps but also nozzles.
{Patent Literature 1}
   Laid-open patent application TOKKAI 2009-274045
{Patent Literature 2}
   Laid-open patent application TOKKAI 2008-264771
{Patent Literature 3}
   Patent No. 4547451
{Patent Literature 4}
   Patent No. 4924907

Another example of a method and an apparatus for generating micro-nano bubbles known from the prior art is described in the document JPS62-295663.

### {SUMMARY OF THE INVENTION}

In conventional technologies, it is extremely difficult to generate a large amount of micro-nano bubbles only with pure water but without using nucleating agent. Granted that addition of a nucleating agent is intended, the agent is required to be used in a significantly reduced amount. In addition, if micro-nano bubbles can be generated in an amount considerably larger than a quantity that conventional technologies will generate, great improvement in washing and sterilization can be expected and, further, the broadening of application of such technique to various usages becomes practicable. Thus, a method for generating micro-nano bubbles by a new technique instead of prior arts and an apparatus for generating micro-nano bubbles capable of actualizing such new technique have been strongly desired.

In the technical field to which the present invention relates, constructing a system capable of generating micro-nano bubbles without metal contamination is still being sought as in the past. As stated above, a prospect that this problem can be solved by employing a compressed-air driven bellows-cylinder pump, in which all the wetted parts thereof are made of fluorine resin, is obtained. Moreover, if micro-nano bubbles can be generated in an amount considerably larger than a conventional quantity using a micro-nano bubble generating apparatus that employs the pump of such configuration, it is expected that such bubble-generating method will be a useful washing method as a response to a demand by technical movements toward the fining of wiring in manufacturing semiconductor devices. At present however, a high performance apparatus of metal-free material that can generate increased amount of bubbles has not been available.

Therefore, it is strongly desired to establish a new method for generating micro-nano bubbles and to develop a high-performance apparatus for generating micro-nano bubbles, by general optimization of structure and shapes of constituents including pumps and other constituting parts such as a bubble generating nozzle, a gas-liquid mixing vessel, and a liquid-feeding device. Ultimately, it is necessary to construct a system capable of generating micro-nano bubbles without metal contamination.

Herein described is an apparatus for generating micro-nano bubbles to construct a system that performs clean washing and sterilizing with large amount of micro-nano bubbles generated using pure water only and can generate micro-nano bubbles without metal contamination. The apparatus intended to be provided, including a bubble generating nozzle and an auto-regulating gas-liquid mixing vessel is to operate on a new method, which is different from conventional arts, for generating micro-nano bubbles using water hammering.

The basic idea for solving the problem described above is use of violent water hammering to generate a large amount of micro-nano bubbles that contains dissolved gas, wherein the water hammering occurs on collision of water, a non-compressive substance. To actualize this, the inventors of the present invention has been led to the present art through, in the method for generating micro-nano bubbles and the apparatus therefor, optimizing the structure and the shape of the bubble generating nozzle so that the water hammering power will work to its utmost extent and constructing an apparatus for generating micro-nano bubbles having a configuration that accelerates generating large amount of micro-nano bubbles. The present invention provides a method and apparatus for generating micro-nano bubbles as set forth in the claims.

The present invention comprises a method for generating micro-nano bubbles as set forth in claim 1. The present invention additionally comprises an apparatus for generating micro-nano bubbles, as set forth in claim 5. Further optional features of the invention are provided in the dependent claims.

The method for generating micro-nano bubbles by the present invention generates micro-nano bubbles using the water hammering power. Therefore, the method is able to generate micro-nano bubbles in a large amount using pure water only without use of substances which are not necessarily needed such as nucleating agents. Accordingly, the method can realize a clean washing and sterilization. Since this water hammering power is maximized by the use of a bubble-generating nozzle having an optimized structure and shape, the use of such optimized nozzle makes it possible to perform continuous and stable generation of bubbles in an efficient manner. Thereby, the amount of generation of small-size bubbles, not only of the size of micrometer order but also of nanometer order, can be increased together. This feature enhances the capability and function in the washing and sterilizing more than those in conventional technique.

The apparatus for generating micro-nano bubbles by the present invention has a bubble generating nozzle and equipment configuration that permits the generating of bubbles in a large amount stably; therefore, the apparatus is usable in a set of equipment for clean washing and sterilizing with pure water.

On the other hand in the gas-liquid mixing vessel that dissolves gas in liquid, transferring gas and liquid in a lump by a pump may sometimes prevent generating uniform micro-nano bubbles. This is because that, if there occurs a phenomenon in which the volume of the gas increases, causing the inside of the gas-liquid mixture vessel to become full of gas and the volume of liquid in the vessel to be lessened, the gas not dissolved in liquid is fed to the bubble generating nozzle in an as-gas-state causing an unstable generation of micro-nano bubbles. This problem is solvable by discharging excess gas from the gas-liquid mixing vessel through a float valve provided inside or outside the vessel. With this float valve, the volume of the gas and the liquid are maintained always within a prescribed range and thereby the amount of generation of the micro-nano bubbles becomes constant.

Further, for a clean washing that is incompatible with metal ion which a wetted part generates, configuring a pump or piping, or both, in a washing apparatus with plastic or preferably with fluorine resin makes the apparatus become to have high reliability and clean feature.

The method and the apparatus by the present invention contribute to constructing a micro-nano bubble generating system that generates bubbles without metal contaminations. For example, application of the present invention to washing such as semiconductor wafers simplifies such washing process compared to conventional processes that perform complicated washing using such as drug solutions. Further, the invention is capable of reducing environmental load because the invented method does not need use of material such as drug solutions. Moreover, the use of the invention for sterilization of foods such as vegetables makes it possible to perform reliable and safe sterilization.

### {BRIEF DESCRIPTION OF THE DRAWINGS}

FIG. 1 is a front view of a micro-nano bubble generation system to which the present invention is applied.
FIG. 2 is a perspective view of the micro-nano bubble generation system to which the present invention is applied.
FIG. 3 is a cross sectional view of a micro-nano bubble generating nozzle.
FIG. 4 is a front view of the micro-nano bubble generating nozzle of figure 3.
FIG. 5 is a side view of the micro-nano bubble generating nozzle of figure 3.
FIG. 6 illustrates a high-speed jet liquid squirting nozzle part.
FIG. 7 is an enlarged partial cross sectional view of the high-speed jet liquid squirting nozzle part of figure 6.
FIG. 8 is a cross sectional view of a nozzle that generates micro-nano bubbles.
FIG. 9 is a cross sectional detail view of the liquid-collision nozzle of figure 8 with which micro-nano bubbles are generated.
FIG. 10 is a front view, a cross sectional view, and a three-dimensional view of the liquid-collision nozzle of figure 8.
FIG. 11 illustrates a liquid-collision nozzle having multi-row of groups of liquid squirting holes.
FIG. 12 is a cross sectional view of a gas-liquid mixing vessel.
FIG. 13 is a detail view of structure of a high-efficiency gas-liquid injection pipe for mixing gas and liquid in the gas-liquid mixing vessel of figure 12.
FIG. 14 is a cross sectional view of a float part to be equipped on the gas-liquid mixing vessel.
FIG. 15 is a cross sectional view of the gas-liquid mixing vessel having a bubble generating nozzle.
FIG. 16 is a cross sectional view of a multi-row nozzle of another configuration that generates micro-nano bubbles.
FIGS. 17A to 17E are a perspective view and a cross sectional view of structures of the liquid-collision nozzles.
FIGS. 18A and 18B are a perspective view and a cross sectional view of structures and shapes of the nozzle cylinders.
FIG. 19 is an illustration that shows the relationship between the flow rate of the jet squirted from the small through-hole of the liquid-collision nozzle and the flow rate of the discharge from the discharge port.
FIG. 20 is a graph that shows schematically the relationship between the diameter of the small through-hole of the liquid-collision nozzle and the number of bubbles per unit volume.
FIG. 21 is a graph that shows the relationship between the diameter of the small through-hole of the liquid-collision nozzle and the flow rate Q at the nozzle.
FIG. 22 is a graph that shows the amount of bubbles generated by the method for generating micro-nano bubbles by the present invention and the particle diameter of the generated bubbles.
FIG. 23 is a graph that shows the amount of bubbles generated by a conventional gas-liquid two-phase swirl flow method and the particle diameter of the generated bubbles.

### {DETAILED DESCRIPTION OF THE PREFERED EMBODIMENTS}

The following describes the best mode for carrying out the present invention, referring to drawings.

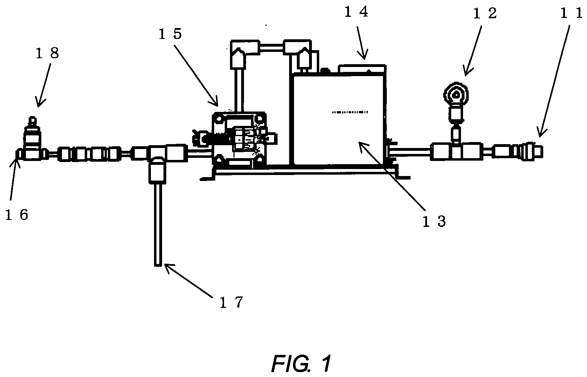

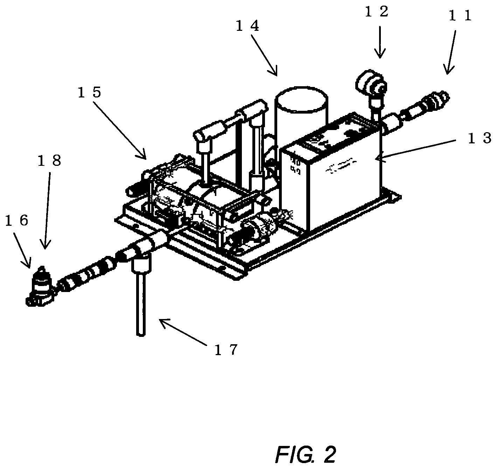

FIG. 1 is a front view of the micro-nano bubble generating system to which the present invention is applied and FIG. 2 is a perspective view of the same. Each of the reference numerals therein denotes;
15: a bellows cylinder pump,
13: a pump controller,
14: a gas-liquid mixing vessel,
12: a pressure sensor,
11: a micro-nano bubble generating nozzle attachment part,
17: a liquid sucking pipe,
16: a gas sucking port, and
18: a gas sucking regulating valve.

These constituents are arranged as illustrated in a perspective view FIG. 2. The bellows cylinder pump 15, wetted parts of which are made of fluorine resin, sucks gas and liquid into the pump in a mixture state through the liquid sucking pipe 17 and the gas sucking regulating valve 18, with the volume of the gas regulated. The sucked liquid and gas mixture is agitated and compressed inside the bellows to dissolve the gas in the liquid. In the present invention, if the bellows cylinder pump 15 has a metal free structure, such configuration is enough. Any plastic other than fluorine resin can also be useable. Usable plastic includes at least one of plastics selected from the group consisting of, for example: general purpose plastics such as polyethylene, polypropylene, and polyethylene terephthalate; engineering plastics such as polyacetar, polyamide, polycarbonate, and denaturing polyphenylene ether; and super engineering plastics such as polyether sulfone, polyphenylene sulfide, polyether ether ketone, and liquid crystal polymer. When use of plastic is intended, applying above stated various plastics including fluorine resin to the wetted part, not only to the pump only, will provide an apparatus for generating micro-nano bubbles having a high reliability and cleanliness. In addition to the above, when washing or sterilizing in a strict metal free condition is not required, metals and ceramics may be used as well as those plastics mentioned above.

Next, the gas and the liquid are agitated by the pump 15 and are force-fed to the gas-liquid mixing vessel 14. The pump 15 used is mainly a bellows cylinder pump of compressed-air driven type but an electric motor driven type may be used. The gas and the liquid in the gas-liquid mixing vessel 14 are under the pressure generated by the pump 15; therefore, the gas is easily dissolved. The pressure that force-feeds the gas and the liquid from the pump 15 is watched by the pressure sensor 12. Increasing the quantity of the dissolved gas with this manner, the preparations are made for increasing the amount of generation of the micro-nano bubbles. In the micro-nano bubble generating system by the present invention, it is a practical manner to use a bellows cylinder pump as the pump 15. Depending on the use purpose however, conventional known pumps are applicable. The applicable pumps include a reciprocating pump such as a piston pump, a plunger pump, or a diaphragm pump; or a rotary pump such as a gear pump, an eccentric pump, or a screw pump.

The liquid entered under force-feeding the gas-liquid mixing vessel 14 mixes with gas to dissolve the gas thereinto and then is transferred to the micro-nano bubble generating nozzle attachment part 11. The micro-nano bubble generating nozzle attachment part 11 is a part to which a nozzle connects, wherein the nozzle generates, from the gas-dissolved liquid, micro-nano bubbles in a large amount having a diameter of 60 µm or smaller, preferably to be 15 µm or smaller.

At that time, the pressure sensor 12 senses variation of the liquid pressure at the section between the micro-nano bubble generating nozzle attachment part 11 and the gas-liquid mixing vessel 14 to watch the dissolving conditions of the gas-liquid. By this, a constant pressure condition needed for stable generation of micro-nano bubbles is actualized.

The process to be performed by the apparatus for generating micro-nano bubbles to which the present invention is applied is as follows. Treatments that the gas sucking port 16, the liquid sucking pipe 17, and the gas sucking regulating valve 18 perform are the gas- and liquid-sucking process. The pressure is regulated by the pressure sensor 12. Next, the gas-including liquid is pressurized using the bellows cylinder pump 15; this treatment is the gas-liquid pressurization process. Following this process, the pressurized gas-including liquid is mixed with another new gas using the pump controller 13 and the gas-liquid mixing vessel 14; this treatment is the dissolved gas enriching process. After this, the bubble generating nozzle by the present invention, which nozzle will be mentioned later, is connected to the micro-nano bubble generating nozzle attachment part 11 to generate micro-nano bubbles. This process is referred to as the dissolved liquid miniaturization process, in which the micro-nano bubbles are generated by injecting the dissolved liquid from the outside of a cylinder, which has two or more small through-holes, via such small through-holes at a pressure higher than the atmospheric pressure to produce jets of the liquid, and the jets are collided mutually at one point inside the cylinder.

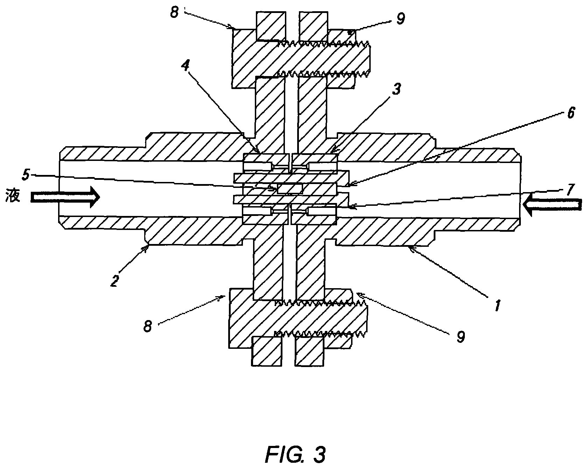

Next, explanation follows to describe a method for generating micro-nano bubbles in a large amount from the dissolved liquid that is in a gas-dissolved state, which is a comparative example useful in understanding the claimed invention but which is not an embodiment thereof. FIG. 3 is a cross sectional view of the micro-nano bubble generating nozzle including an attachment part. In the figure, reference numerals 1 and 2 are the outer cases of the nozzle, which are disposed facing each other and fastened together using a bolt 8 and a nut 9, wherein the dissolved liquid force-fed by the pump is divided into two streams and each of the streams is separately introduced into the outer cases 1 and 2 as arrows show. In each of the outer cases so disposed, a recess is provided to accommodate the high-speed liquid jet squirting nozzle parts 3 and 4. The size of the nozzle hole determines the flow rate and the flow velocity of the liquid discharge.

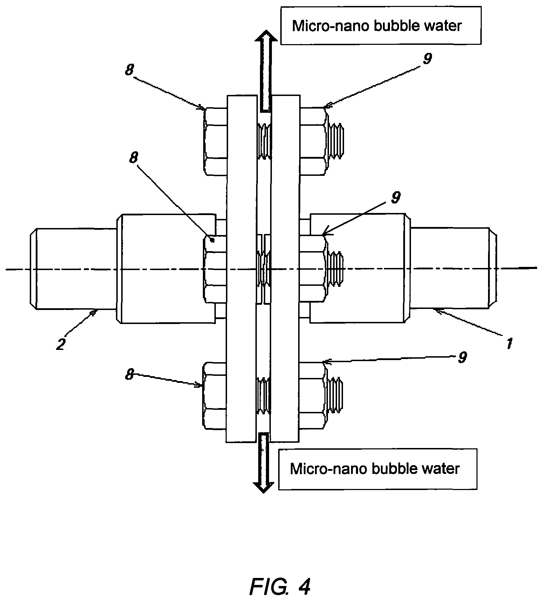

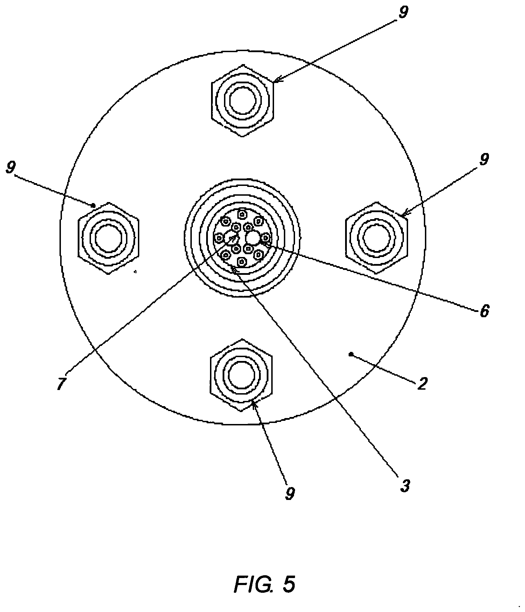

FIG. 4 illustrates the nozzle in the fabricated state, in which the bolt 8 is tightening-fastened by the nut 9. The water that produced micro-nano bubble is discharged in the radial direction indicated by arrows. FIG. 5 is a side view of the micro-nano bubble generating nozzle by high-speed liquid jet squirting, in which the micro-nano bubble water is discharged in the circumferential direction.

The following explains how to generate the micro-nano bubbles using the water stream squirted from the high-speed liquid jet squirting nozzle. The gas-dissolved liquid is discharged from the high-speed liquid jet squirting nozzle at the discharge pressure of 0.2 MPa to 0.6 MPa given by the high-pressure pump 15. The discharged liquid rapidly releases its pressure and collides violently each other producing a water hammering power. The explosive water hammering smashes the gas-dissolve liquid and makes the liquid to be in a state that a large amount of micro-nano bubbles is involved therein. It should be noted that, depending on the method of release, there is a case where the amount of generation of micro-nano bubbles becomes reduced. However, the micro-nano bubbles can be generated in a large amount with the method and the apparatus by the present invention.

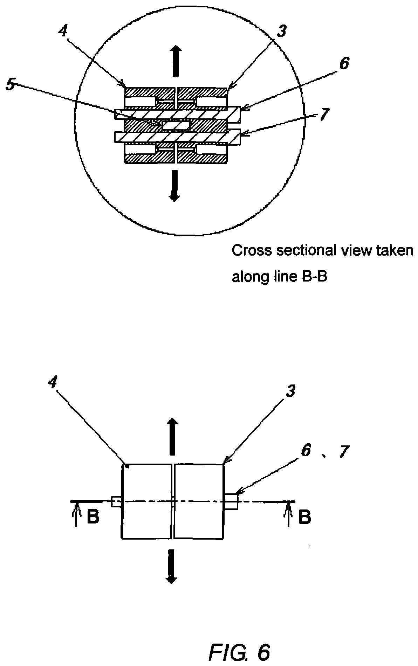

FIG. 6 is a cross sectional view taken along the line B-B and an outline view of the high-speed liquid jet squirting nozzle parts 3 and 4 that generate micro-nano bubbles. The high-speed liquid jet squirting nozzle parts 3 and 4 are, as illustrated in the cross sectional view, centered relying on the centering pin 5, which defines the center, and positioned guided by the positioning pins 6 and 7, and then fixed. The high-speed liquid jet squirting nozzle parts 3 and 4 generate the micro-nano bubbles.

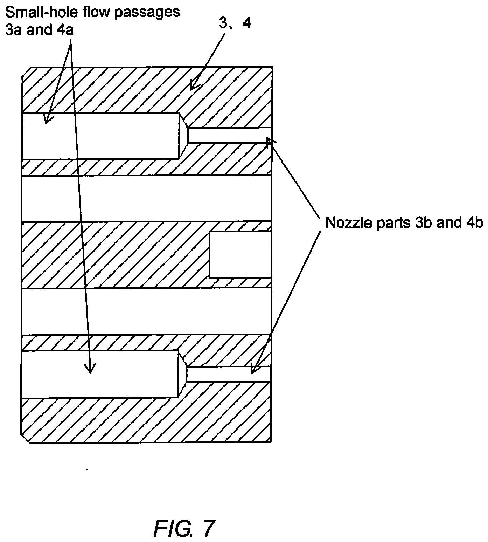

FIG. 7 is an enlarged cross sectional view of the high-speed liquid jet squirting nozzle parts 3 and 4 illustrated in FIG. 6. Since these parts 3 and 4 have the same shape and disposed symmetrically, the following explains using reference numerals 3 and 4 for simplicity. To feed the gas-dissolved liquid to 3 and 4 in high-speed jets, the liquid stream is narrowed at small-hole flow passages 3a and 4a. Thereby the jet stream squirts from the end of small-hole flow passages 3a and 4a that narrow the liquid stream. Nozzle parts 3b and 4b are arranged so that the jets squirted from each of the high-speed liquid jet squirting nozzle parts 3 and 4 collide, producing micro-nano bubbles from the collided gas-dissolved liquid. The micro-nano bubbles that involve gas inside diffuse in the circumferential direction indicated by arrows.

The reason of feeding the liquid at a high-pressure is to increase the speed of the liquid in squirting from the small-hole. This means that making the liquid collision high-speed increases the impact energy and that a large amount of micro-nano bubbles of more minute size can be generated thereby.

Assume that F is the power of collision. Also assume that the density of a liquid is p, the size of a small-hole S, and the velocity of a liquid V. Then, the relationship of F = ρSV² holds. For the optimal value of F, the optimum design that considers the relation between the size of hole S and the velocity V is needed.

What is important here is that, if a pump capable of generating a higher pressure is used, there is a possibility that further-large amount of micro-nano bubbles can be generated. For example, it is available to use a high-pressure pump that generates pressures of 0.5 to 250 MPa or so. If a pump of this kind is used, the liquid velocity V increases proportionally to the pressure and the amount of generation of micro-nano bubbles greatly increases because the impact power of the water hammering power F increases with the square of V. However, for the application of such high-pressure pump to an apparatus for generating micro-nano bubbles, it is difficult to meet various demands such as light weight, small size, metal-free, and low maintenance cost.

In the present invention, by using the nozzle having a structure as illustrated in FIGS. 3 to 7, the amount of generation of micro-nano bubbles can be made equal to or more than the conventional quantity when the pressure of squirting the dissolved liquid of the gas-liquid mixing state is the atmospheric pressure (about 0.1 MPa) or more. Further, setting the pressure to 0.2 MPa or higher makes it possible to generate micro-nano bubbles in an amount sufficient for performing clean washing and sterilizing. As mentioned above in the present invention, the lower limit of the squirting pressure of the dissolved liquid can be set to 0.2 MPa, a lower value than the conventional pressure. It therefore becomes practicable to use a pump suitable for eliminating metal contamination, that is, the compressed-air driven or electric-motor driven bellows cylinder pump 15 made of fluorine resin, as shown in FIGS. 1 and 2. On the other hand, if the squirting pressure of the dissolved liquid exceeds 0.6 MPa while using the compressed-air driven or electric-motor driven bellows cylinder pump of the present invention, the amount of generation of micro-nano bubbles tends to be saturated. Therefore, the squirting pressure of the dissolved liquid in the present invention is preferred to be 0.2 to 0.6 MPa.

The micro-nano bubble generating nozzle by the present invention needs to have a diameter of 0.1 to 6 mm at its nozzle parts 3b and 4b shown in FIG. 7 so as to squirt the jet of the dissolved liquid in a pressure higher than the atmospheric pressure, preferably 0.2 to 0.6 MPa, which are lower than conventional pressures. Here, the openings of the nozzle parts 3b and 4b, from which the jet stream squirts, and the diameters of the nozzle parts 3b and 4b correspond to the "spout" and the "diameter of the small through-holes of the nozzle at the part leading to the hollow of the cylinder" respectively, as the present invention defines. The reason for specifying the diameter of the nozzle parts 3b and 4b to be 0.1 to 6 mm will be detailed in the description of embodiment example later.

The small-hole flow passages 3a and 4a are enough when they are such a device as has a stream-narrowing function for feeding the gas-dissolved liquid in a form of a high-speed jet; and when they are taper-shaped continuously toward the nozzle parts 3b and 4b, they may also be enough. The amount of generation of micro-nano bubbles is determined mainly by the dimension of the diameter of the nozzle parts 3b and 4b; therefore, the small-hole flow passages 3a and 4a may be omitted in the present invention.

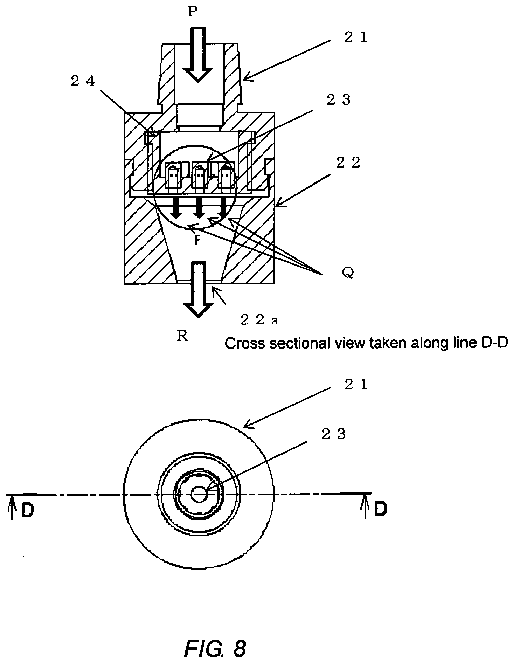

An example of another method for colliding the gas-dissolved liquid will be explained referring to FIG. 8. Illustrated in FIG. 8 is the nozzle for generating micro-nano bubbles, which is comprised of a nozzle case 21, a micro-nano bubble discharging nozzle 22, and a base 24, wherein one or more liquid-collision nozzles 23 are installed on the base 24.

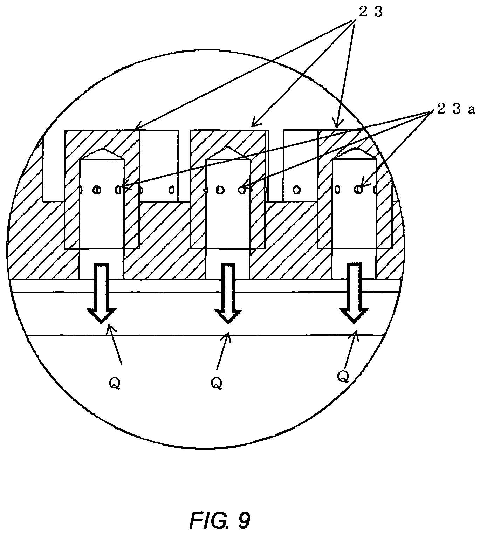

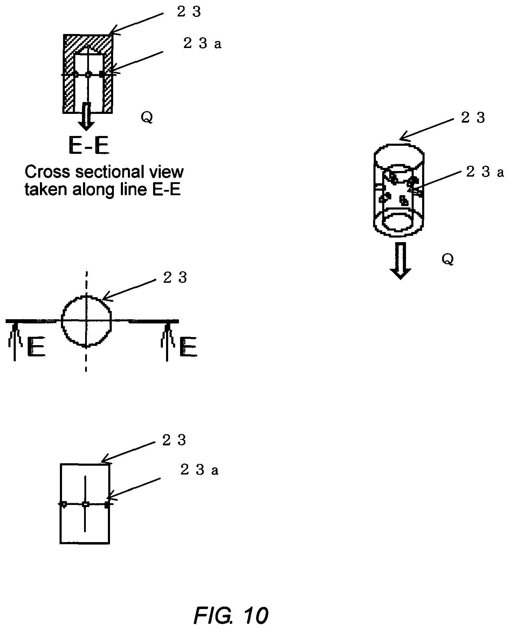

FIG. 9 is an enlarged view of the part where the liquid-collision nozzle 23 shown in FIG. 8 is disposed. FIG. 10 illustrates the shape of a single piece of the liquid-collision nozzle 23. A small-hole 23a opens toward the center of the liquid-collision nozzle 23. High-pressure liquid entering through this small-hole at a high-pressure in the direction of the arrow Q collides in the center of the liquid-collision nozzle 23, generating micro-nano bubbles.

The experiment told that controlling the velocity of liquid V made the amount of generated micro-nano bubbles increased and prolonged the life of bubbles. When the velocity V exceeds 25 m/s as a guideline, the nozzle generates micro-nano bubbles stably.

The same effect will be obtained at a lower liquid velocity by squirting the liquid toward center from every direction concentrating the water hammering at the center. This means that when water hammering is given from every direction, the same or more effect will be produced even if the velocity is reduced to 1/2. For example, since F = 2ρSV², when eight holes are arranged so that the hammering among the jets concentrates in the center, the force at the center becomes F = ρS(1/2)² x 8 = 2ρSV². Thus, when the small-hole of the nozzle is provided in a plural number for concentrating the water hammering produced by the liquid collision, the energy of the liquid collision becomes same even if the velocity V is low because the flowing quantity of liquid increases. Since the amount of generation of micro-nano bubbles will be acceptably same if the energy in the collisions of the liquid are same, the pressure of discharging the liquid can be lowered and the amount of generation of micro-nano bubbles will be secured as desired.

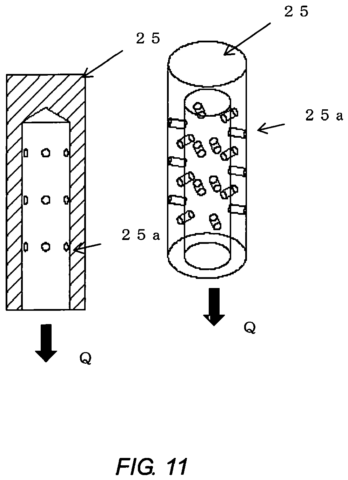

FIG. 10 illustrates the shape of the liquid-collision nozzles 23, wherein a small-hole 23a is made in the circumference of the nozzle-cylinder part of the liquid-collision nozzle 23. Through this small-hole, the dissolved liquid squirts to collide in the center and generates micro-nano bubbles. The micro-nano bubbles thus generated is discharged toward the arrow Q. When a plurality of the liquid-collision nozzle 23 is gather-arrayed, a large amount of micro-nano bubbles are generated and are discharged from a nozzle part 22a of the discharging nozzle 22 illustrated in FIG. 8. As illustrated in FIG. 11, the shape of a liquid-collision nozzle 25 is such a shape as has small-holes 25a in a multi-row configuration. For example therefore, when three places are provided for producing the water hammering by making holes in a three-row configuration, it becomes practicable to generate micro-nano bubbles in a large amount. Thus, this practice is a useful method for miniaturization and increased efficiency.

Discharging the liquid from a plurality of holes, as the nozzle illustrated in FIG. 11, increases the intensity of the water hammering. Using this technique will not decrease the amount of generation of micro-nano bubbles even if the velocity V is low; and consequently a pump with a high discharging pressure is not needed, which imposes less defrayment. Thus, this technique is industrially very useful and permits development of a nozzle having good efficiency.

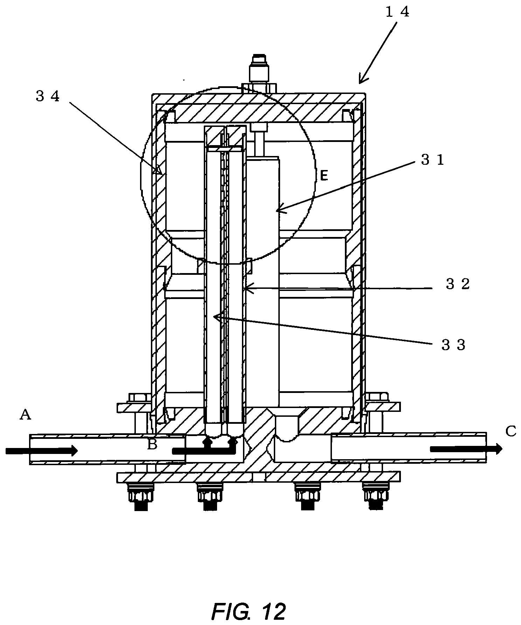

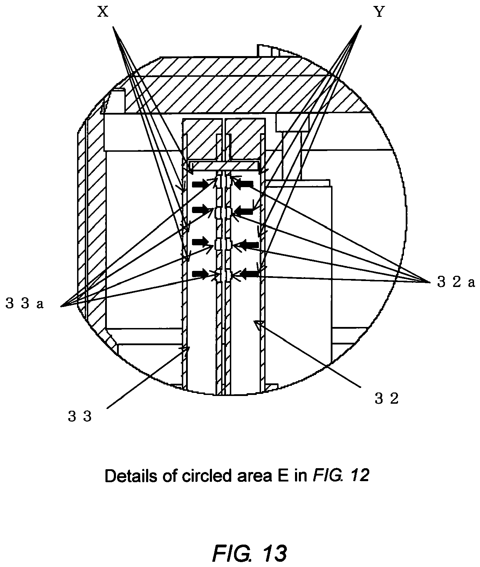

FIG. 12 is a cross sectional view of the gas-liquid mixing vessel 14. FIG. 13 is an enlarged view of the circled area E in FIG. 12. In a conventional gas-liquid mixing vessel, wherein gas and liquid are mixed under a high pressure, the mixture of gas and pure water is fed into the gas-liquid mixing vessel by a pump and spouted therein upward like a water spout to merge the mixture. This method however is not efficient in mixing; therefore it is necessary to generate micro-nano bubbles in an increased amount for improved efficiency.

Then, as illustrated in FIG. 12, gas and liquid are fed by a pump in the direction of the arrow A toward the arrow B and are introduced into gas-liquid injection pipes 32 and 33. And as FIG. 13 illustrates, water hammering produced by colliding liquid in the directions from the arrow X and the arrow Y is used for increased efficiency of the mixing of the gas and liquid discharged from a hole of gas-liquid injection pipe 32a and a hole of gas-liquid injection pipe 33a of the gas-liquid injection pipe 33. Thereby, mixing gas and liquid is performed efficiently and gas-liquid mixture liquid as a raw material of micro-nano bubbles is produced speedily with increased merging rate.

A float 31 illustrated in FIG. 12 is provided for the purpose of exhausting excessively entered gas to outside if gas enters excessively in mixing gas and liquid. The float 31 exhausts entered excess gas safely and regulates the amount of gas and liquid to a proper level. This means that a certain situation is produced as described below. If there remains undissolved gas due to the entering of excessive amount of gas, the gas will flow into the nozzle and impedes the generation of micro-nano bubbles. The float eliminates this adverse effect of impeding bubble generation and controls the amount of generation of the micro-nano bubbles to a proper level, enabling sending bubbles stably.

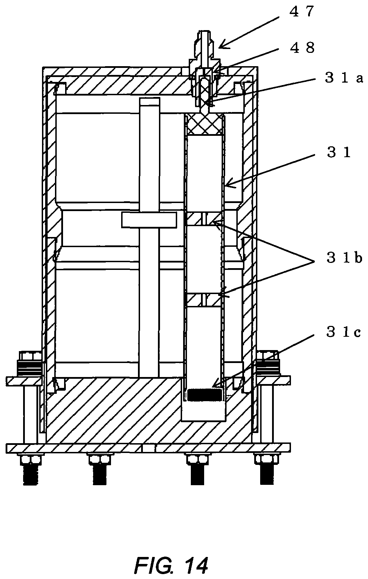

FIG. 14 illustrates a cross sectional view of a part of the float 31 shown in FIG. 12. The structure of the float pipe 31 comprises a float tip part 31a (which is tapered toward its end), a reinforcing rib 31b for prevention of collapse by the pressure of liquid, and a stop plug 31c.

Mixing gas and liquid requires its method to increase the dissolving efficiency of gas into liquid by enlarging the contact area of gas and liquid. If the efficiency lowers, shortage of the generation amount occurs due to a shortage of gas, which is a fatal problem in the generating of micro-nano bubbles.

We examined how much the amount of generated micro-nano bubbles will be increased depending on the degree of control over the amount of gas and liquid. As a result, it was understood that the following are the points. If the amount of liquid in the volume ratio inside the gas-liquid mixing vessel occupies 60% and the amount of gas occupies 40%, the gas-liquid ratio is the ideal balance of amounts. To stabilize the amount of generation of the micro-nano bubbles and to increase the amount of generated bubbles, it is necessary that the condition of the mixing of gas to be dissolved and liquid should be optimized by exhausting the excess gas from an excess gas exhausting port 48 of a float socket 47 using the buoyancy of the float 31 caused by the liquid for controlling their ratio automatically. In the present invention, for the purpose of increasing greatly the amount of generation of micro-nano bubbles, it is preferable to control the volume ratio of gas and liquid in the gas-liquid mixing vessel within the range of gas to liquid ratio = 50 : 50 to 5 : 95 so that liquid will occupy more part in the volume ratio. Also in the present invention, the float 31 may be installed outside the gas-liquid mixing vessel, instead of installing inside. In this arrangement, connecting the inside and outside of the gas-liquid mixing vessel using such as a communication pipe permits controlling the volume ratio of gas and liquid.

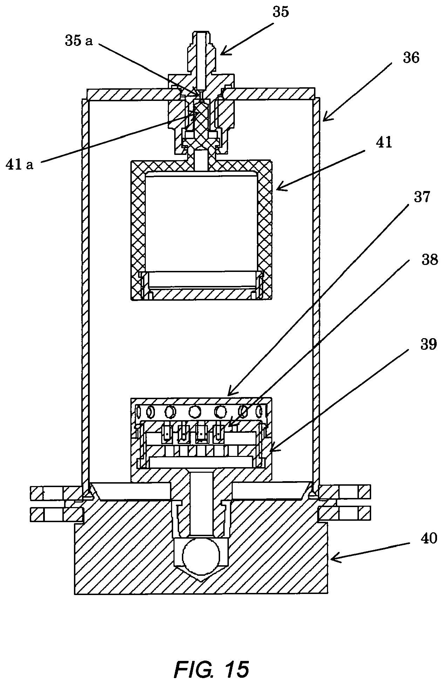

FIG. 15 is an example of a gas-liquid mixing vessel that has more improved efficiency. The fundamental working is same as those illustrated in FIGS. 12 and 13. This vessel is a gas-liquid mixing vessel structured based on a gas-liquid mixing vessel comprising a gas-liquid mixing vessel outer frame 36, a micro-nano bubble generating nozzle 38, and a member 40, wherein a structure resistible to the internal pressure of the gas-liquid mixing vessel is given to the basic structure. The mixing of gas and liquid is efficiently performed inside that vessel.

In FIG. 15, each of the reference numerals denotes:
36: the gas-liquid mixing vessel outer frame;
41: a float;
35: a float holder;
35a: an excess gas exhausting port on float holder; and
41a: a float tip, wherein the float tip 41a has a function that regulates automatically the excess gas.

When using an conventional apparatus that generates a less amount of micro-nano bubbles, a major method for generating an increased amount of micro-nano bubbles is as follows. The method is comprised of processes of: generating micro-nano bubbles once in a water tank; pumping up micro-nano bubbles generated in the water tank again; injecting additional gas to be dissolved into the pumped bubble-containing liquid at the gas-liquid mixing vessel; and circulating the gas-injected bubble-containing liquid multiple times to bring the bubble-containing liquid to a state in which a large amount of micro-nano bubbles are involved. Thereby, micro-nano bubbles are generated in an increased amount.

In this method, it is difficult to control the amount of generation of the micro-nano bubbles. Further, circulating technique invites a trouble such as occurrence of contamination. Because of that, an apparatus that is capable of generating a large amount of micro-nano bubbles in one process without use of circulation technique is desired.

Therefore, it is intended to generate micro-nano bubbles, without circulation in the gas-liquid mixing vessel to be used in the present invention, by a liquid-collision in the gas-liquid mixture state under the working of the micro-nano bubble generating nozzle 38, which is held on a nozzle holder 39, having a structure same as illustrated in FIG. 8.

In this situation, it is the requisite condition that the nozzle 38 arranged inside the gas-liquid mixing vessel should issue the dissolved liquid of the gas-liquid mixture state at a flow rate more than that of the nozzle 11 arranged at the distal end to increase the pressure inside the gas-liquid mixing vessel. If the flow rate of the nozzle 38 is smaller, micro-nano bubbles sometimes may not be generated from the nozzle attached at the distal end.

The effect of the installing of the nozzle 38 inside the gas-liquid mixing vessel is that one-path of processing along the gas-liquid mixing vessel to the nozzle permits a stable generation of a large amount of micro-nano bubbles. Thereby, such technique enables provision of an apparatus suitable for washing process in semiconductor manufacturing line for example.

In the present invention, configuring gas-liquid mixing vessels in a multi-stage cascade makes it possible to generate a larger amount of micro-nano bubbles; this is a useful means for generating a large amount of bubbles.

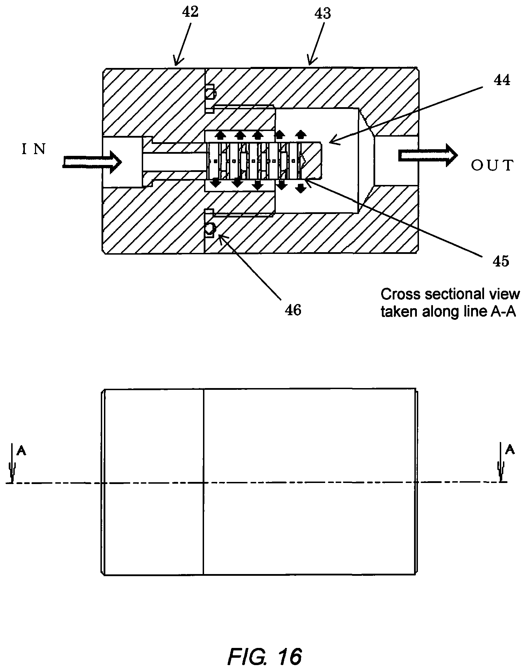

FIG. 16 offers another method for generating micro-nano bubbles, which is a comparative example useful in understanding the claimed invention but which is not an embodiment thereof. This method employs an arrangement in which two or more small-hole nozzles 45 are arrayed perpendicular to the incoming or discharging direction (longitudinal direction) of the flow of the dissolved liquid. This array is different from the parallel arrangement illustrated in FIG. 8. The arrangement illustrated in FIG. 16 has an advantage in that a desired flow rate is easily ensured, because the small-hole nozzles are arrayed as the figure shows with respect to the outer cases 42 and 43, a packing 46, and a nozzle hole 44; and thereby the small-hole nozzles become to have discharging ports on their both sides.

As can be seen in FIG. 16, the gas-liquid mixture liquid fed from the port IN is brought into a water hammering state by the nozzle 45 to generate micro-nano bubbles, and then discharged to the both sides of the nozzle. This ensures the flow rate as desired and the efficiency is doubled, therefore the energy needed for generating micro-nano bubbles becomes half.

Bubbles like this produced by a water hammering gives less damage to the nozzle structure, because collision occurs only between the liquid. Therefore, it is possible to make a bubble generating apparatus have a longer service life.

The significant feature of the method and the apparatus for generating micro-nano bubbles by the present invention is that they are compatible with using pure water as a dissolved liquid that does not include any foreign matters such as nucleating agent in an application to washing and sterilization of semiconductor devices and food. Granted that a use of nucleating agent, or the like, is needed to increase the amount of generation of micro-nano bubbles, the quantity of addition of such material into pure water can be considerably reduced. In the present invention, tap water, well water, or spring water such as natural water other than pure water can be used in consideration of the supply state or usability. Further in the present invention, the strengthening of the oxidizing action of the dissolved liquid and the reformulating of the liquid for enhancing permeability required for impurity removing action may be practicable to increase the effectiveness of the washing and sterilizing.

The method for strengthening the oxidizing action of the dissolved liquid stated above includes the use of the dissolved liquid which is an aqueous solution prepared by adding, to pure water, at least one of oxidant selected from the group consisting of ozone, oxygen, hydrogen peroxide, chloric acid, perchloric acid, and potassium permanganate. Among these oxidant, ozone and oxygen are preferable oxidant for the present invention, because they have little adverse effect as an additive and their environmental load is very small.

As the method for enhancing the permeability for impurity removing action in the dissolved liquid stated above, it is a preferable method to add a gas selected from the group consisting of carbon dioxide, hydrogen gas, and nitrogen gas, which has excellent permeability for impurity removing action. On generation of micro-nano bubbles, carbon dioxide, hydrogen gas, or nitrogen gas invades easily the boundary surface between a semiconductor device and impurities, such as residuals of resist, adhering to its surface. Thereby, the effectiveness of the washing is largely increased. Further, since carbon dioxide or nitrogen gas is harmless to human body, such gas is suitable for the present invention as a reformulating additive.

The structure and shape of the micro-nano bubble generating nozzle by the present invention will be detailed referring to concrete embodiments.

### {First Embodiment}

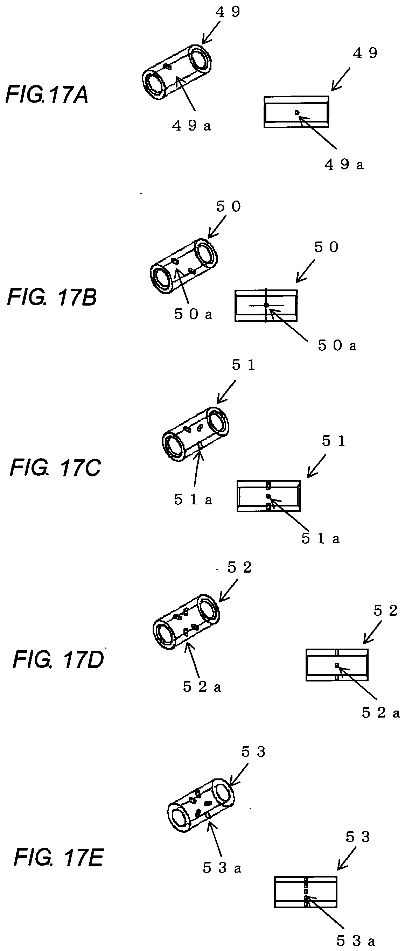

FIG. 17 illustrates the structure and shape of the liquid-collision nozzle which was examined to generate micro-nano bubbles using a water hammering power. FIG. 17A illustrates a comparative example to the embodiment of the present invention and FIGS. 17B to 17E illustrate embodiments of the present invention.

FIG. 17A illustrates a configuration that a single-hole 49a is made in the circumference of a hollow cylinder. In this comparative example, which has the single-hole 49a of one small through-hole, the dissolved liquid squirted at a speed V collides against the wall of a pipe 49. Therefore, the amount of generated micro-nano bubbles is not much and there is a disadvantage in that the impact by the collision against the wall may damage the wall.

A liquid-collision nozzle illustrated in FIG. 17B has a two-hole 50a configured with two small through-holes. In this embodiment, the dissolved liquid squirting at a speed V actualizes a collision at a speed of 2V, because the two-hole 50a provides another spout at the opposite side. Thus, the collision energy becomes higher than that of the comparison example of FIG. 17A.

A liquid-collision nozzle illustrated in FIG. 17C has a three-hole 51a configured with three small through-holes. In this embodiment, the dissolved liquid squirts at a speed V from three holes provided at an interval of 120 degree for collision. Thereby, the energy in the center of collision of the liquid squirted at a speed V becomes three times. This means that, when the collision is produced using three spouts and when the collision energy same as the one in the former two-hole case is considered to be enough, the collision produces still the same amount of energy even if the speed V is reduced by as much as 20%. Since the pump pressure determines the speed V, even a lowered pump pressure is still capable of generating micro-nano bubbles.

A liquid-collision nozzle illustrated in FIG. 17D has a four-hole 52a configured with four small through-holes. In this embodiment, the dissolved liquid squirts at a speed V from four holes provided at an interval of 90 degree for collision. Thereby, the energy in the center of collision of the liquid squirted at a speed V becomes four times. This means that, when the collision is produced using four spouts and when the collision energy same as the one in the former two-hole case is considered to be enough, the collision produces still the same amount of energy even if the speed V is reduced by as much as 30%. Since the pump pressure determines the speed V, even a lowered pump pressure is still capable of generating micro-nano bubbles.

A liquid-collision nozzle illustrated in FIG. 17E has a five-hole 53a configured with five small through-holes. In this embodiment, the dissolved liquid squirts at a speed V from five holes provided at an interval of 72 degree for collision. Thereby, the energy in the center of collision of the liquid squirted at a speed V becomes five times. This means that, when the collision is produced using five spouts and when the collision energy same as the one in the former two-hole case is considered to be enough, the collision produces still the same amount of energy even if the speed V is reduced by as much as 40%. Since the pump pressure determines the speed V, even a lowered pump pressure is still capable of generating micro-nano bubbles.

As stated above, micro-nano bubbles, which were not generated without a high-pressure pump, can be generated in a large amount by optimizing the structure and arrangement of the through-hole of the liquid-collision nozzle even if the pump pressure is 0.2 MPa; thus this technique is able to actualize energy-saving.

### { Second Embodiment}

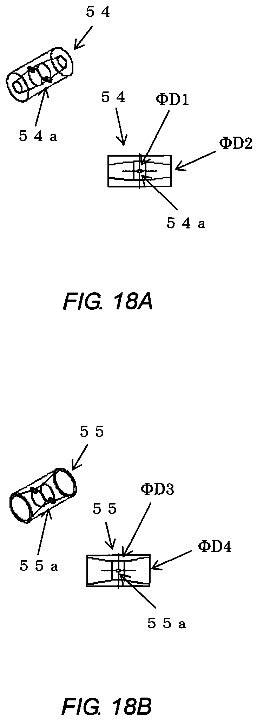

Referring to FIG. 18, the following explains a diameter-related feature in the liquid-collision nozzle by the present invention that generates micro-nano bubble. The explanation describes the relationship between the diameter of a micro-nano bubble discharging port to be provided on the end of a hollow cylinder and the diameter of the hollow cylinder at its part where a through-hole is arranged in the circumferential direction.

FIG. 18A illustrates a liquid-collision nozzle having two holes 54a. In a nozzle cylinder 54, the diameter of the part (the part in which the small through-hole is made) against which the jet of liquid collides is made large, referred to as D1, and the diameter of a discharging port is made small, referred to as D2. With this configuration, pressure is immediately imposed on micro-nano bubbles generated by the collision. Thereby, the composition of micro-nano bubbles can be controlled. That is, this diameter configuration works as a means for controlling the distribution of particles in micro-nano bubbles and therefore an advantageous effect is brought in the miniaturization of particles.

A liquid-collision nozzle illustrated in FIG. 18B has two holes 55a. In a nozzle cylinder 55, the diameter of the part against which the jet of liquid collides is made small, referred to as D3, and the diameter of a discharging port is made large, referred to as D4. With this configuration, pressure is not imposed on micro-nano bubbles generated by the collision. Therefore, the distribution of particles of micro-nano bubbles which expand on generation becomes a little large.

In the present invention, either of the nozzles having the structure illustrated in FIG. 18 may be used. The nozzle cylinder shown in FIG. 18A has such a structure that the diameter of the discharging port is smaller than the diameter of the collision occurring part of the nozzle cylinder; therefore, the pressure of the dissolved liquid in the vicinity of the spout becomes high. Because of that, even though there is an advantageous effect for miniaturization of micro-nano bubble particles, the bubble generation is a little bit impeded. As a consequence to this, the lowering of the amount of generated bubbles, or the late-generating of bubbles at a place away from the spout, may occur. In contrast to this, the nozzle having the structure illustrated in FIG. 18B is able to generate a large amount of bubbles stably, because the nozzle allows pressure release at its discharging port. Since it is possible to regulate the miniaturization of micro-nano bubbles by the diameter of the small through-hole of the nozzle, the nozzle illustrated in FIG. 18B is suitable.

### {Third Embodiment}

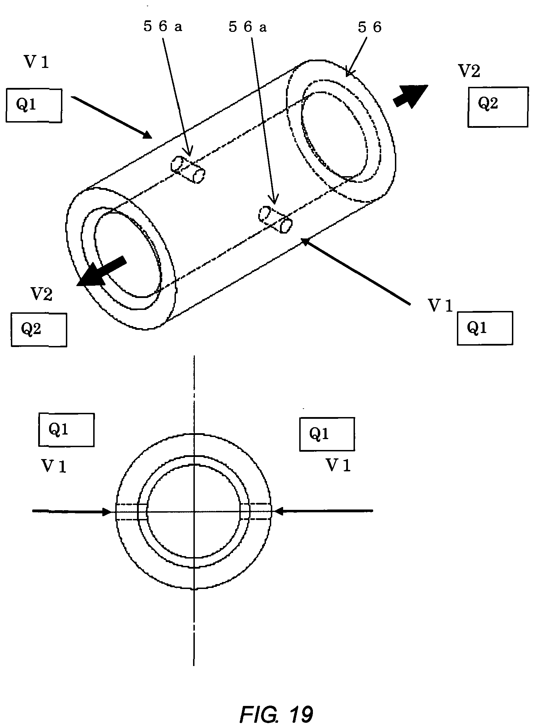

Regarding a liquid-collision nozzle illustrated in FIG. 18B, the relationship between the diameter of the liquid-collision nozzle and micro-nano bubbles is explained referring to FIGS. 19 to 21, wherein the pressure is kept constant and pure water, which includes 50 ppm of ozone, is used as the dissolved liquid.

FIG. 19 illustrates the relationship between the flow rate of the jet squirted from the small through-hole of a nozzle 56 and the flow rate of the discharge issued from the discharge port. In FIG. 19, the dissolved liquid squirted from a small through-hole 56a at a speed VI, after its squirting from a single-hole 56a at a liquid rate Q1 per second, collides to generate micro-nano bubbles. Then, it goes out from the discharge port of the nozzle 56 at a speed V2 with a liquid rate Q2 per second. Here, the flow rates Q1 and Q2 are same.

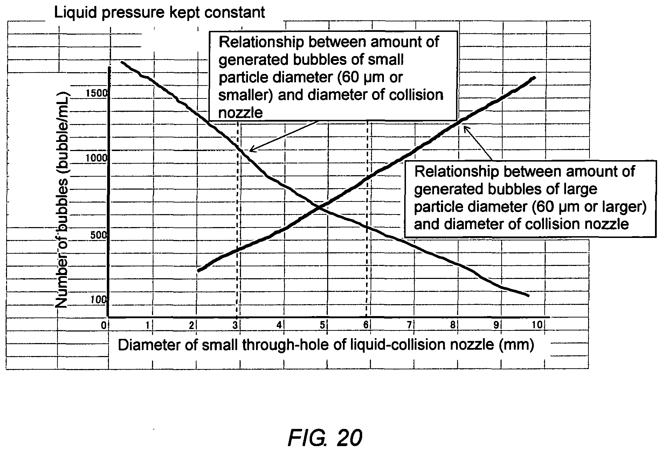

FIG. 20 shows the relationship between the diameter of the small through-hole of the liquid-collision nozzle and the amount of generation of micro-nano bubbles. In this graph, the amount of generated micro-nano bubbles is plotted in the number of bubbles generated per unit volume of the dissolved liquid. As can be known from FIG. 20, when the diameter of the small through-hole of the liquid-collision nozzle becomes larger, V1 reduces and the liquid rate Q1 increases. In this case, the amount of generated micro bubbles (60 µm or larger in diameter) increases but the amount of generated nano bubbles reduces. On the other hand, when the diameter of a liquid-collision nozzle becomes smaller, the liquid rate Q1 reduces. In this case, the amount of generated micro bubbles (60 µm or larger in diameter) reduces and V1 increases, but the amount of generated nano bubbles (2 µm or smaller in diameter) increases.

Thus, the diameter of the small through-hole of the liquid-collision nozzle is an important factor that determines the performance of the micro-nano bubbles. Although there is a difference in behavior depending on the nature of the liquid and the gas to be dissolved, the tendency is as described in the above. Therefore, the amount of micro-nano bubbles can be controlled by adjusting the diameter of the small through-hole of the liquid-collision nozzle.

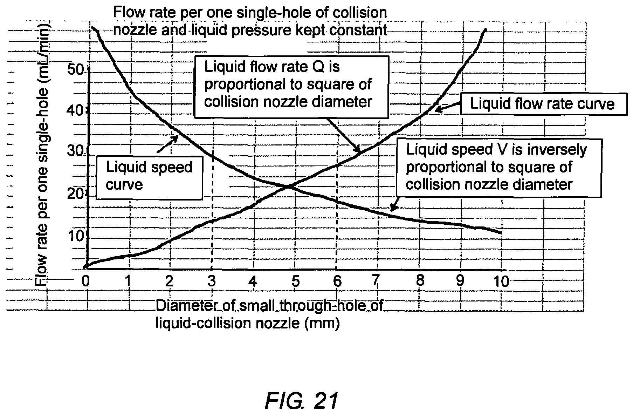

FIG. 21 shows the relationship between the diameter of the small through-hole of the liquid-collision nozzle and the flow rate Q. Where the liquid pressure is kept constant, the flow rate Q is proportional to the square of the diameter of the small through-hole of the liquid-collision nozzle, but the liquid speed V is inversely proportional to the square of the diameter of the liquid-collision nozzle. Thus, the relationship between the diameter of one single-hole of the small through-hole of the liquid-collision nozzle and the flow rate Q is as shown in FIG. 20. The optimization of the diameter of the small through-hole of the nozzle is possible by determining the number of holes of the liquid-collision nozzles in view of the required flow rate.

As can be known from FIG. 20, it is necessary in the present invention that the small through-hole of the nozzle should have a diameter of 0.1 to 6.0 mm. If the diameter of the small through-hole of the nozzle is smaller than 0.1 mm, the amount of generation of small-size bubbles of about 60 µm or smaller in particle diameter increases. However, the amounts of generation of bubbles having larger diameters than that particle diameter decreases sharply; then, micro-nano bubbles are hardly generated. In addition, if the diameter of the small through-hole of the nozzle is in excess of 6 mm, the total amount of generated bubbles increases. On the contrary however, the amount of generation of small-size bubbles of about 60 µm or smaller in particle diameter decreases sharply to 500 bubbles per mL or less; thus, it is not possible to achieve a sufficient effect of the present invention. It is more preferable in the present invention that the diameter of the small through-hole of the liquid-collision nozzle should be configured within a range of 0.1 to 3 mm to generate micro-nano bubbles in a large amount of 1000 bubbles per mL or more.

### {Fourth Embodiment}

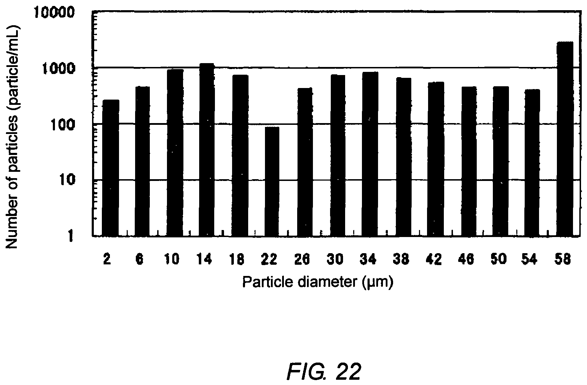

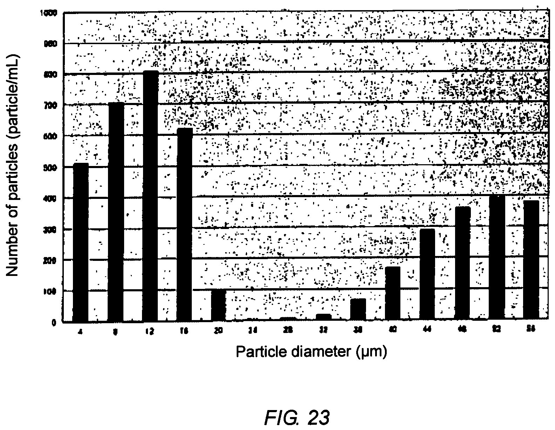

Micro-nano bubbles were generated using distilled water as the dissolved liquid with the apparatus for generating micro-nano bubbles by the present invention as illustrated in FIGS. 1 and 2. The nozzle used had the same structure as illustrated in FIGS. 3 and 4, and the nozzle parts 3b and 4b were given a straight shape of a small through-hole of 0.5 mm in diameter. In addition, as a comparison example, micro-nano bubbles were generated by the conventional gas-liquid two-phase swirl flow method using, similarly to the above, distilled water as the dissolved liquid. FIGS. 22 and 23 show the relationship between the amount of generated bubbles and the particle diameter of bubbles. FIG. 22 shows the amount generated by the method for generating micro-nano bubbles by the present invention and FIG. 23 shows the same using the gas-flow two-phase swirl flow method. The amount of generated bubbles is indicated in the number of bubbles per unit volume of the distilled water (bubble/mL). The amount of generated bubbles and their particle diameter are determined using a submerged particle counter at room temperature. FIGS. 22 and 23 do not show the amount of bubbles having particle diameters in the nano region; this is because of the difficulty in measuring the number of particles in the nano region with an optical method.

Comparison of the results shown in FIGS. 22 and 23 teaches that the method for generating micro-nano bubbles by the present invention generates larger amount of bubbles compared to the amount generated by the conventional gas-liquid two-phase swirl flow method over the entire particle diameter region of about 60 µm or smaller. Particularly, the difference in the region of the bubble particle diameter of 20 to 40 µm is considerable. In addition, comparison of both methods for the region of the bubble particle diameter of 2 to 10 µm tells that the generated bubbles by the present invention is equal to or slightly larger than that by the conventional method in the amount. By analogy from the results of the number of generated bubbles in the region of a small particle diameter, the present invention can be considered to be a method that generates a large number of bubbles in the sub-micron region, i.e., even in the nano region. A use of the apparatus for generating micro-nano bubbles by the present invention for the washing semiconductor wafers and the sterilization of food such as vegetables actually has confirmed that the effect of washing or sterilization continued even when the dissolved liquid became an apparent-clear solution over time, like a bubble-disappeared liquid. This means that the present invention gains an effect of being able to continue rendering a washing and sterilization effect by generating micro-nano bubbles, for a long time more than the conventional method would provide. It is understood that the existence of nano-bubbles, which is large in amount, having smaller diameter of less than 1 µm has produced such an effect.

As stated above, the method for generating micro-nano bubbles by the present invention generates micro-nano bubbles using the water hammering power. Therefore, the method is able to generate micro-nano bubbles in a large amount using pure water only without use of substances which are not necessarily needed such as nucleating agents. Accordingly, the method can realize a clean washing and sterilization. Since this water hammering power is maximized by the use of a bubble generating nozzle having an optimized structure and shape and by an apparatus that is able to stably perform the generation of a large amount of bubbles, the application of such combination makes it possible to perform continuous and stable generation of bubbles in an efficient manner. Thereby, the amount of generation of small-size bubbles, not only of the size of micrometer order but also of nanometer order, can be increased together. This feature enhances the capability and function in the washing and sterilizing more than those in conventional technique.

Further, for a clean washing that is incompatible with metal ion which a wetted part generates, configuring a pump or piping, or both, in a washing apparatus with plastic or preferably with fluorine resin makes the apparatus become to have high reliability and clean feature. Thus, the apparatus for generating micro-nano bubbles by the present invention is applicable to the clean washing for such as semiconductor wafers. Conventionally, the washing of semiconductor wafers has used processing with such as strong acid treatment, alkaline neutralization, and pure water rinsing. The process therefore has been complicated and the environmental load has been large because, for example, the process uses drug solutions. However, the present invention is able to solve this problem. Further, the process burden such as in the disposal of drug solutions is eliminated and the required scale for semiconductor manufacturing equipment becomes small and related process is made compact; these are a great industrial value.

Further, in the semiconductor wafer washing, the use of a micro-nano bubble-generated liquid, other than pure water, improves the washing effect largely and makes the washing process very simple with the washing equipment downsized. The micro-nano bubble-generated liquid for such use is prepared by adding a gas having excellent oxidizing ability like oxygen or a permeable impurity removal agent such as carbon dioxide or nitrogen gas, and then followed by the micro-nano bubble generation process by the present invention. Thereby, the washing becomes environment friendly. Further, the process burden such as in the disposal of drug solutions is eliminated and the required scale for semiconductor manufacturing equipment becomes small and related process is made compact; these are a great industrial value.

The micro-nano bubble generating system by the present invention is applicable to medical use, because the system uses micro-nano bubbles generated by a clean system that uses pumps and wetted part made of fluorine resin. Therefore, it is expected that the field of the application of the invented system will expand greatly.

Further, the capability of the washing and sterilization by micro-nano bubbles that uses oxygen or ozone as its constitution gas can be applied not only to the semiconductor field but also to fields of foods and vegetables. Thus, there is a possibility in that the application range may expand to the fields such as agriculture and the fishery; and the method for generating micro-nano bubbles, the bubble generating nozzle, and the apparatus for generating micro-nano bubbles by the present invention have a very high superiority in such field expansion movement.

## Claims

1. A method for generating micro-nano bubbles, wherein the method uses water hammering power to generate micro-nano bubbles, having a diameter of 60µm or smaller, wherein micro-nano bubbles are generated by a mutual collision of jets of a dissolved liquid of a gas-liquid mixture state; the collision of jets is created inside a hollow cylinder (23) having two or more small through-holes (23a) with a diameter of 0.1 to 6.0 mm, arrayed in the circumferential direction thereof with such a configuration that the respective openings of such two or more small through-holes (23a) are arranged facing each other in the same plane; and the jets of the dissolved liquid are produced by injecting the liquid from the outside of the hollow cylinder (23) via the small through-holes (23a) in the hollow cylinder (23) at a pressure higher than the atmospheric pressure;
wherein the dissolved liquid of a gas-liquid mixture state is prepared by a method comprising:
a sucking process that sucks gas and liquid;
a pressurization process that pressurizes gas and liquid; and
a dissolved gas enriching process that mixes the pressurized gas-including liquid with another new gas,
wherein, in order to essentially maintain an inflow direction of the dissolved liquid unchanged until reaching the inlet position of the small through-holes (23a), the hollow cylinder (23) having such two or more small through-holes (23a) arrayed in the circumferential direction thereof is arranged in parallel to the inflow direction of the dissolved liquid
wherein the hollow cylinder (23) further has one end formed into a closed tube-like shape and an outer wall with a smaller cross sectional area than that of an introduction opening of the dissolved liquid, which faces the outer wall of the closed end formed into the closed tube-like shape, wherein the hollow cylinder (23) is provided inside a nozzle case (21) for feeding the dissolved liquid to the hollow cylinder (23); and
wherein two or more hollow cylinders (23) are aligned in parallel, in a plane perpendicular to the inflow direction.

2. The method for generating micro-nano bubbles according to claim 1, wherein the pressure of being more than the atmospheric pressure at the time of squirting is 0.2 to 0.6 MPa, and the diameter of the small through-holes at the part leading to the hollow of the cylinder is 0.1 to 3.0 mm.

3. The method for generating micro-nano bubbles according to any of claims 1 to 2, wherein the dissolved liquid is an aqueous solution that includes at least one of substance selected from the group consisting of ozone, oxygen, hydrogen peroxide, chloric acid, perchloric acid, and potassium permanganate.

4. The method for generating micro-nano bubbles according to any of claims 1 to 2, wherein the dissolved liquid is an aqueous solution that includes a substance selected from the group consisting of carbon dioxide, hydrogen gas, and nitrogen gas.

5. An apparatus for generating micro-nano bubbles, comprising
a means (16, 17, 18) for sucking each of gas and liquid;
a means (15) for pressurizing the gas and the liquid in a lump and transferring them;
a gas-liquid mixing vessel (14) for enriching the dissolved gas by mixing the transferred liquid, which includes the gas, with another new gas; and
a means for generating micro-nano bubbles using the dissolved liquid of the gas-liquid mixing state prepared in the gas-liquid mixing vessel (14), wherein the means has a bubble generating nozzle for generating micro-nano bubbles, having a diameter of 60 µm or smaller, using water hammering power caused by a mutual collision of the dissolved liquid,
wherein the bubble generating nozzle comprises:
a hollow cylinder (23) having two or more small through-holes (23a) with a diameter of 0.1 to 6.0 mm, arrayed in the circumferential direction thereof with such a configuration that the respective opening of each of such two or more small through-holes (23a) faces each other in the same plane; and a micro-nano bubble discharge port provided on at least one end of the hollow cylinder,
wherein the small through-holes (23a) are arranged so that all of their extension lines passing through respective center of the cross-section of each of the small through-holes (23a) intersect each other in the inside of the hollow cylinder (23);
wherein, in order to essentially maintain the inflow direction of the dissolved liquid unchanged until reaching the inlet position of the small through-holes (23a), the hollow cylinder (23) having such two or more small through-holes (23a) arrayed in the circumferential direction thereof is arranged in parallel to the inflow direction of the dissolved liquid,
wherein the hollow cylinder (23) further has one end formed into a closed tube-like shape and an outer wall with a smaller cross sectional area than that of an introduction port of the dissolved liquid, which faces the outer wall of the closed end formed into the closed tube-like shape,
wherein the hollow cylinder (23) is provided inside a nozzle case (21) for feeding the dissolved liquid to the hollow cylinder (23); and
wherein two or more hollow cylinders (23) are aligned in parallel, in a plane perpendicular to the inflow direction.

6. The apparatus for generating micro-nano bubbles according to claim 5, wherein the diameter of the small through-hole at the part that leads to the hollow of the hollow cylinder is 0.1 to 3.0 mm.

7. The apparatus for generating micro-nano bubbles according to any of claims 5 or 6, wherein the diameter of the micro-nano bubble discharge port provided on at least one end of the hollow cylinder (23 or 45) is equal to or larger than the diameter of a part of the hollow cylinder, wherein such part is such a part where the small through-holes are arranged in a circumferential direction.

8. The apparatus for generating micro-nano bubbles according to any of claims 5 to 7, wherein, in the means for generating micro-nano bubbles, the dissolved liquid is squirted at a pressure of 0.2 to 0.6 MPa through the small through-hole of the bubble generating nozzle.

9. The apparatus for generating micro-nano bubbles according to any of claims 5 to 8, wherein the gas-liquid mixing vessel (14) has the bubble generating nozzle for generating micro-nano bubbles, and the liquid that includes the gas transferred by the means for pressurizing and transferring is discharged into the gas-liquid mixing vessel (14) by the bubble generating nozzle.

10. The apparatus for generating micro-nano bubbles according to any of claims 5 to 9, wherein the gas-liquid mixing vessel (14) has a float valve inside or outside the vessel to maintain the volume of the gas and the liquid and the internal pressure inside the vessel always within a prescribed range by discharging excess gas from the vessel.

11. The apparatus for generating micro-nano bubbles according to any of claims 5 to 10, wherein a pump (15) or piping (17), or both, through which the gas-liquid mixture liquid flows, is made of plastic.

12. The apparatus for generating micro-nano bubbles according to claim 11, wherein a pump (15) or piping (17), or both, through which the gas-liquid mixture liquid flows, is made of fluorine resin.

13. The apparatus for generating micro-nano bubbles according to any of claims 5 to 12, wherein the means for pressurizing and transferring the liquid that includes the gas is an apparatus that uses a compressed-air driven or an electric motor driven bellows cylinder pump (15).

14. The apparatus for generating micro-nano bubbles according to any of claims 5 to 13, wherein the dissolved liquid is an aqueous solution that includes at least one of substance selected from the group consisting of ozone, oxygen, hydrogen peroxide, chloric acid, perchloric acid, and potassium permanganate.

15. The apparatus for generating micro-nano bubbles according to any of claims 5 to 14, wherein the dissolved liquid is an aqueous solution that includes a substance selected from the group consisting of carbon dioxide, hydrogen gas, and nitrogen gas.

## Patentansprüche

1. Verfahren zur Erzeugung von Mikro-Nano-Blasen, wobei das Verfahren Wasserschlagleistung verwendet, um Mikro-Nano-Blasen mit einem Durchmesser von 60 µm oder weniger zu erzeugen, wobei Mikro-Nano-Blasen durch eine gegenseitige Kollision von Strahlen einer gelösten Flüssigkeit eines Gas-Flüssigkeitsgemisch-Zustandes erzeugt werden; die Kollision der Strahlen im Inneren eines Hohlzylinders (23), der zwei oder mehrere kleine Durchgangslöcher (23a) mit einem Durchmesser von 0,1 bis 6,0 mm aufweist, erzeugt wird, die in Umfangsrichtung desselben angeordnet sind, und zwar mit einer solchen Konfiguration, dass die jeweiligen Öffnungen von zwei oder mehreren solchen kleinen Durchgangslöchern (23a) in derselben Ebene einander gegenüberliegend angeordnet sind; und wobei die Strahlen der gelösten Flüssigkeit hergestellt werden, indem die Flüssigkeit von außerhalb des Hohlzylinders (23) mittels der kleinen Durchgangslöcher (23a) in den Hohlzylinder (23) bei einem Druck, der höher als atmosphärischer Druck ist, injiziert wird;
wobei die gelöste Flüssigkeit eines Gas-Flüssigkeitsgemisch-Zustandes durch ein Verfahren hergestellt wird, das Folgendes umfasst:
einen Ansaugvorgang, der Gas und Flüssigkeit ansaugt;
einen Druckerzeugungsvorgang, der Gas und Flüssigkeit unter Druck setzt; und
einen Gelöstes-Gas-Anreicherungsvorgang, der die unter Druck gesetzte, Gas umfassende Flüssigkeit mit einem weiteren neuen Gas vermischt,
wobei, um eine Einströmrichtung der gelösten Flüssigkeit im Wesentlichen unverändert zu belassen, bis die Einlassposition der kleinen Durchgangslöcher (23a) erreicht wird, der Hohlzylinder (23), der zwei oder mehrere solche kleinen Durchgangslöcher (23a) aufweist, die in Umfangsrichtung desselben angeordnet sind, parallel zur Einströmrichtung der gelösten Flüssigkeit angeordnet ist;
wobei der Hohlzylinder (23) ferner ein Ende, das in Form ähnlich eines geschlossenen Rohrs gebildet ist, und eine Außenwand mit einer kleineren Querschnittsfläche als jener einer Einbringungsöffnung der gelösten Flüssigkeit, die der Außenwand des geschlossenen Endes, das in Form ähnlich eines geschlossenen Rohrs gebildet ist, gegenüberliegt, aufweist, wobei der Hohlzylinder (23) im Inneren eines Düsengehäuses (21) zur Zufuhr der gelösten Flüssigkeit in den Hohlzylinder (23) bereitgestellt ist; und
wobei zwei oder mehrere Hohlzylinder (23) parallel, in einer Ebene, die normal auf die Einströmrichtung steht, ausgerichtet sind.

2. Verfahren zur Erzeugung von Mikro-Nano-Blasen nach Anspruch 1, wobei der Druck, der größer ist als der atmosphärische Druck, zum Zeitpunkt des Spritzens 0,2 bis 0,6 MPa beträgt und der Durchmesser der kleinen Durchgangslöcher an dem Abschnitt, der zum Hohlraum des Zylinders führt, 0,1 bis 3,0 mm beträgt.

3. Verfahren zur Erzeugung von Mikro-Nano-Blasen nach einem der Ansprüche 1 bis 2, wobei die gelöste Flüssigkeit eine wässrige Flüssigkeit ist, die zumindest eine Substanz, die aus der aus Ozon, Sauerstoff, Wasserstoffperoxid, Chlorsäure, Perchlorsäure und Kaliumpermanganat bestehenden Gruppe ausgewählt ist, umfasst.

4. Verfahren zur Erzeugung von Mikro-Nano-Blasen nach einem der Ansprüche 1 bis 2, wobei die gelöste Flüssigkeit eine wässrige Flüssigkeit ist, die zumindest eine Substanz, die aus der aus Kohlendioxid, Wasserstoffgas und Stickstoffgas bestehenden Gruppe ausgewählt ist, umfasst.

5. Vorrichtung zur Erzeugung von Mikro-Nano-Blasen, die Folgendes umfasst:
ein Mittel (16, 17, 18) zum Ansaugen von sowohl Gas als auch Flüssigkeit;
ein Mittel (15) zum Unter-Druck-Setzen und Transportieren von Gas und Flüssigkeit auf einmal;
ein Gas-Flüssigkeitsmischgefäß (14) zum Anreichen des gelösten Gases durch das Mischen der transportierten Flüssigkeit, die das Gas umfasst, mit einem neuen Gas; und
ein Mittel zum Erzeugen von Mikro-Nano-Blasen unter Verwendung der gelösten Flüssigkeit des Gas-Flüssigkeitsmisch-Zustandes, der in dem Gas-Flüssigkeitsmischgefäß (14) hergestellt wurde, wobei das Mittel eine blasenerzeugende Düse zur Herstellung der Mikro-Nano-Blasen mit einem Durchmesser von 60 µm oder weniger, unter Verwendung von Wasserschlagleistung, die durch gegenseitige Kollision von Strahlen einer gelösten Flüssigkeit hervorgerufen wird, umfasst;
wobei die blasenerzeugende Düse Folgendes umfasst:
einen Hohlzylinder (23) mit zwei oder mehr kleinen Durchgangslöchern (23a) mit einem Durchmesser von 0,1 bis 6,0 mm, die in Umfangsrichtung desselben angeordnet sind, mit einer solchen Konfiguration, dass die jeweilige Öffnung jedes der zwei oder mehrerer solcher kleinen Durchgangslöcher (23a) in derselben Ebene gegenüberliegt; und eine Mikro-Nano-Blasen-Auslassöffnung, die auf zumindest einem Ende des Hohlzylinders bereitgestellt ist,
wobei die kleinen Durchgangslöcher (23a) so angeordnet sind, dass alle ihre Verlängerungslinien, die durch die jeweilige Mitte des Querschnitts jedes der kleinen Durchgangslöcher (23a) verlaufen, sich im Inneren des Hohlzylinders (23) schneiden;
wobei, um eine Einströmrichtung der gelösten Flüssigkeit im Wesentlichen unverändert zu belassen, bis die Einlassposition der kleinen Durchgangslöcher (23a) erreicht wird, der Hohlzylinder (23), der zwei oder mehrere solche kleinen Durchgangslöcher (23a) aufweist, die in Umfangsrichtung desselben angeordnet sind, parallel zur Einströmrichtung der gelösten Flüssigkeit angeordnet wird;
wobei der Hohlzylinder (23) ferner ein Ende, das in Form ähnlich eines geschlossenen Rohrs gebildet ist, und eine Außenwand mit einer kleineren Querschnittsfläche als jener der Einbringungsöffnung der gelösten Flüssigkeit, die der Außenwand des geschlossenen Endes, das in Form ähnlich eines geschlossenen Rohrs gebildet ist, gegenüberliegt, aufweist,
wobei der Hohlzylinder (23) in einem Düsengehäuse (21) zur Zufuhr der gelösten Flüssigkeit in den Hohlzylinder (23) bereitgestellt ist; und
wobei zwei oder mehrere Hohlzylinder (23) parallel, in einer Ebene, die normal auf die Einströmrichtung steht, ausgerichtet sind.

6. Vorrichtung zur Erzeugung von Mikro-Nano-Blasen nach Anspruch 5, wobei der Durchmesser des kleinen Durchgangsloches an dem Abschnitt, der zum Hohlraum des Hohlzylinders führt, 0,1 bis 3,0 mm beträgt.

7. Vorrichtung zur Erzeugung von Mikro-Nano-Blasen nach Anspruch 5 oder 6, wobei der Durchmesser der Mikro-Nano-Blasen-Auslassöffnung, die auf zumindest einem Ende des Hohlzylinders (23 oder 45) bereitgestellt ist, gleich wie oder größer als der Durchmesser eines Abschnitts des Hohlzylinders ist, wobei so ein Abschnitt ein solcher Abschnitt ist, in dem die kleinen Durchgangslöcher in Umfangsrichtung angeordnet sind.

8. Vorrichtung zur Erzeugung von Mikro-Nano-Blasen nach einem der Ansprüche 5 bis 7, wobei in dem Mittel zur Erzeugung von Mikro-Nano-Blasen die gelöste Flüssigkeit in einem Druck von 0,2 bis 0,6 MPa durch das kleine Durchgangsloch der blasenerzeugenden Düse gespritzt wird.

9. Vorrichtung zur Erzeugung von Mikro-Nano-Blasen nach einem der Ansprüche 5 bis 8, wobei das Gas-Flüssigkeitsmischgefäß (14) die blasenerzeugende Düse zur Erzeugung von Mikro-Nano-Blasen aufweist, und die Flüssigkeit, die das Gas umfasst, das von dem Mittel zum Unter-Druck-Setzen und Transportieren transportiert wird, von der blasenerzeugenden Düse in das Gas-Flüssigkeitsmischgefäß (14) ausgestoßen wird.

10. Vorrichtung zur Erzeugung von Mikro-Nano-Blasen nach einem der Ansprüche 5 bis 9, wobei das Gas-Flüssigkeitsmischgefäß (14) ein Schwimmventil innerhalb oder außerhalb des Gefäßes aufweist, um das Volumen von Gas und Flüssigkeit und den Innendruck innerhalb des Gefäßes immer in einem vorgeschriebenen Bereich zu halten, indem überschüssiges Gas aus dem Gefäß ausgestoßen wird.

11. Vorrichtung zur Erzeugung von Mikro-Nano-Blasen nach einem der Ansprüche 5 bis 10, wobei ein(e) Pumpe (15) oder Rohrsystem (17) oder beides, durch die/das die Gas-Flüssigkeitsgemisch-Flüssigkeit strömt, aus Kunststoff hergestellt ist.

12. Vorrichtung zur Erzeugung von Mikro-Nano-Blasen nach Anspruch 11, wobei ein(e) Pumpe (15) oder Rohrsystem (17) oder beides, durch die/das die Gas-Flüssigkeitsgemisch-Flüssigkeit strömt, aus Fluorharz hergestellt ist.

13. Vorrichtung zur Erzeugung von Mikro-Nano-Blasen nach einem der Ansprüche 5 bis 12, wobei das Mittel zum Unter-Druck-Setzen und Transportieren der Flüssigkeit, die das Gas umfasst, eine Vorrichtung ist, die eine Druckluftbetriebene oder Elektromotor-betriebene Balgzylinderpumpe (15) verwendet.

14. Vorrichtung zur Erzeugung von Mikro-Nano-Blasen nach einem der Ansprüche 5 bis 13, wobei die gelöste Flüssigkeit eine wässrige Flüssigkeit ist, die zumindest eine Substanz, die aus der aus Ozon, Sauerstoff, Wasserstoffperoxid, Chlorsäure, Perchlorsäure und Kaliumpermanganat bestehenden Gruppe ausgewählt ist, umfasst.

15. Vorrichtung zur Erzeugung von Mikro-Nano-Blasen nach einem der Ansprüche 5 bis 14, wobei die gelöste Flüssigkeit eine wässrige Flüssigkeit ist, die zumindest eine Substanz, die aus der aus Kohlendioxid, Wasserstoffgas und Stickstoffgas bestehenden Gruppe ausgewählt ist, umfasst.

## Revendications

1. Procédé de génération de microbulles et nanobulles, dans lequel le procédé utilise une force de type coup de bélier pour générer des microbulles et nanobulles, ayant un diamètre de 60 µm ou moins, dans lequel les microbulles et nanobulles sont générées par une collision mutuelle de jets d'un liquide dissous dans un état de mélange gaz-liquide ; la collision de jets est créée à l'intérieur d'un cylindre creux (23) possédant deux petits trous traversants (23a) ou plus ayant un diamètre de 0,1 à 6,0 mm, disposés dans la direction circonférentielle de celui-ci avec une configuration telle que les ouvertures respectives desdits deux petits trous traversants (23a) ou plus sont agencées face à face dans le même plan ; et les jets du liquide dissous sont produits en injectant le liquide depuis l'extérieur du cylindre creux (23) via les petits trous traversants (23a) dans le cylindre creux (23) à une pression supérieure à la pression atmosphérique ;
dans lequel le liquide dissous dans un état de mélange gaz-liquide est préparé par un procédé comprenant :
un processus d'aspiration qui aspire le gaz et le liquide ;
un processus de mise sous pression qui met sous pression le gaz et le liquide ; et
un processus d'enrichissement en gaz dissous qui mélange le liquide incluant le gaz sous pression avec un autre gaz nouveau ;
dans lequel, afin de maintenir essentiellement une direction de flux entrant du liquide dissous inchangée jusqu'à atteindre la position d'entrée des petits trous traversants (23a), le cylindre creux (23) possédant lesdits deux petits trous traversants (23a) ou plus disposés dans la direction circonférentielle de celui-ci est agencé en parallèle à la direction de flux entrant du liquide dissous,
dans lequel le cylindre creux (23) possède en outre une extrémité formée en une forme de type tube fermé et une paroi externe ayant une surface en section transversale plus petite que celle d'une ouverture d'introduction du liquide dissous, qui est face à la paroi externe de l'extrémité fermée formée en la forme de type tube fermé, dans lequel le cylindre ceux (23) est prévu à l'intérieur d'un carter de buse (21) pour fournir le liquide dissous au cylindre creux (23) ; et
dans lequel deux cylindre creux (23) ou plus sont alignés en parallèle, dans un plan perpendiculaire à la direction de flux entrant.

2. Procédé de génération de microbulles et nanobulles selon la revendication 1, dans lequel
la pression étant supérieure à la pression atmosphérique au moment de l'injection est de 0,2 à 0,6 MPa et le diamètre des petits trous traversants au niveau de la partie conduisant au creux du cylindre est de 0,1 à 3,0 mm.

3. Procédé de génération de microbulles et nanobulles selon l'une quelconque des revendications 1 et 2, dans lequel
le liquide dissous est une solution aqueuse qui inclut au moins une substance choisie dans le groupe constitué d'ozone, d'oxygène, de péroxyde d'hydrogène, d'acide chlorique, d'acide perchlorique et de permanganate de potassium.

4. Procédé de génération de microbulles et nanobulles selon l'une quelconque des revendications 1 et 2, dans lequel
le liquide dissous est une solution aqueuse qui inclut une substance choisie dans le groupe constitué de dioxyde de carbone, d'hydrogène gazeux et d'azote gazeux.

5. Appareil de génération de microbulles et nanobulles, comprenant
un moyen (16, 17, 18) pour aspirer chacun du gaz et du liquide ;
un moyen (15) pour mettre sous pression le gaz et le liquide en bloc et les transférer ;
un récipient de mélange gaz-liquide (14) pour enrichir le gaz dissous en mélangeant le liquide transféré, qui inclut le gaz, avec un autre gaz nouveau ; et
un moyen pour générer des microbulles et nanobulles grâce au liquide dissous dans l'état de mélange gaz-liquide préparé dans le récipient de mélange gaz-liquide (14), dans lequel le moyen possède une buse de génération de bulles pour générer des microbulles et nanobulles, ayant un diamètre de 60 µm ou moins, grâce à une force de type coup de bélier causée par une collision mutuelle du liquide dissous,
dans lequel la buse de génération de bulles comprend :
un cylindre creux (23) possédant deux petits trous traversants (23a) ou plus ayant un diamètre de 0,1 à 6,0 mm, disposés dans la direction circonférentielle de celui-ci avec une configuration telle que l'ouverture respective de chacun desdits deux petits trous traversants (23a) ou plus est en face de l'autre dans le même plan ; et un orifice d'évacuation de microbulles et nanobulles prévu dans au moins une extrémité du cylindre creux,
dans lequel les petits trous traversants (23a) sont agencés de telle sorte que toutes leurs lignes d'extension passant à travers le centre respectif de la section transversale de chacun des petits trous traversants (23a) se coupent à l'intérieur du cylindre creux (23) ;
dans lequel, afin de maintenir essentiellement la direction de flux entrant du liquide dissous inchangée jusqu'à atteindre la position d'entrée des petits trous traversant (23a), le cylindre creux (23) possédant lesdits deux petits trous traversants (23a) ou plus disposés dans la direction circonférentielle de celui-ci est agencé en parallèle à la direction de flux entrant du liquide dissous,
dans lequel le cylindre creux (23) possède en outre une extrémité formée en une forme de type tube fermé et une paroi latérale ayant une surface en section transversale plus petite que celle d'un orifice d'introduction du liquide dissous, qui fait face à la paroi externe de l'extrémité fermée formée en la forme de type tube fermé,
dans lequel le cylindre creux (23) est prévu à l'intérieur d'un carter de buse (21) pour fournir le liquide dissous au cylindre creux (23) ; et
dans lequel deux cylindre creux (23) ou plus sont alignés en parallèle, dans un plan perpendiculaire à la direction de flux entrant.

6. Appareil de génération de microbulles et nanobulles selon la revendication 5, dans lequel le diamètre du petit trou traversant au niveau de la partie qui conduit au creux du cylindre creux est de 0,1 à 3,0 mm.

7. Appareil de génération de microbulles et nanobulles selon l'une quelconque des revendications 5 ou 6, dans lequel le diamètre de l'orifice d'évacuation de microbulles et nanobulles prévu sur au moins une extrémité du cylindre creux (23 ou 45) est supérieur ou égal au diamètre d'une partie du cylindre creux, dans lequel ladite partie est une partie où les petits trous traversants sont agencés dans une direction circonférentielle.

8. Appareil de génération de microbulles et nanobulles selon l'une quelconque des revendications 5 à 7, dans lequel, dans le moyen pour générer des microbulles et nanobulles, le liquide dissous est injecté à une pression de 0,2 à 0,6 MPa à travers le petit trou traversant de la buse de génération de bulles.

9. Appareil de génération de microbulles et nanobulles selon l'une quelconque des revendications 5 à 8, dans lequel le récipient de mélange gaz-liquide (14) possède la buse de génération de bulles pour générer des microbulles et nanobulles, et le liquide qui inclut le gaz transféré par le moyen de mise sous pression et de transfert est évacué dans le récipient de mélange gaz-liquide (14) par la buse de génération de bulles.

10. Appareil de génération de microbulles et nanobulles selon l'une quelconque des revendications 5 à 9, dans lequel le récipient de mélange gaz-liquide (14) possède une soupape à flotteur à l'intérieur ou l'extérieur du récipient pour maintenir le volume du gaz et du liquide et la pression interne à l'intérieur du récipient toujours à l'intérieur d'une plage prédéfinie en évacuant l'excès de gaz du récipient.

11. Appareil de génération de microbulles et nanobulles selon l'une quelconque des revendications 5 à 10, dans lequel une pompe (15) ou une conduite (17), ou les deux, à travers lesquelles s'écoule le liquide de mélange gaz-liquide, est en plastique.

12. Appareil de génération de microbulles et nanobulles selon la revendication 11, dans lequel une pompe (15) ou une conduite (17), ou les deux, à travers lesquelles s'écoule le liquide de mélange gaz-liquide, est en résine fluorée.

13. Appareil de génération de microbulles et nanobulles selon l'une quelconque des revendications 5 à 12, dans lequel le moyen pour mettre sous pression et transférer le liquide qui inclut le gaz est un appareil qui utilise une pompe à cylindre à soufflet (15) entraînée par air comprimé ou entraînée par moteur électrique.

14. Appareil de génération de microbulles et nanobulles selon l'une quelconque des revendications 5 à 13, dans lequel le liquide dissous est une solution aqueuse qui inclut au moins une substance choisie dans le groupe constitué d'ozone, d'oxygène, de péroxyde d'hydrogène, d'acide chlorique, d'acide perchlorique et de permanganate de potassium.

15. Appareil de génération de microbulles et nanobulles selon l'une quelconque des revendications 5 à 14, dans lequel le liquide dissous est une solution aqueuse qui inclut une substance choisie dans le groupe constitué de dioxyde de carbone, d'hydrogène gazeux et d'azote gazeux.
